# EUROPEAN PATENT SPECIFICATION

(11) **EP 4 225 951 B1**
(45) Date of publication and mention of the grant of the patent: **21.01.2026**
(21) Application number: 21878094.8
(22) Date of filing: 07.10.2021
(51) Int. Cl.: C12Q 1/689

(54) **SET OF PRIMERS, COMPOSITION OF REAGENTS AND METHOD OF DETECTING ATYPICAL BACTERIA**
PRIMERSATZ, REAGENZIENZUSAMMENSETZUNG UND VERFAHREN ZUM NACHWEIS ATYPISCHER BAKTERIEN
ENSEMBLE D'AMORCES, COMPOSITION DE RÉACTIFS ET PROCÉDÉ DE DÉTECTION DE BACTÉRIES ATYPIQUES

(30) Priority: 09.10.2020 PL 43563420
(43) Date of publication of application: 16.08.2023
(73) Proprietor: Genomtec S.A., 51-317 Wroclaw (PL)
(72) Inventor: TOKARSKI, Miron, 49-300 Brzeg (PL); PIELKA, Izabela, 42-274 Konopiska (PL); MALODOBRA-MAZUR, Malgorzata, 51-354 Wroclaw (PL)
(74) Representative: Witek, Rafal
(86) International application number: PCT/PL2021/050071
(87) International publication number: WO 2022/075871

(56) References cited:
- WO-A1-2017/103269
- WO-A1-2017/103269
- WO-A1-2018/089942
- WO-A1-2018/089942
- WO-A2-2015/063498
- CN-A- 111 455 075
- SOMBOONNA NARAPORN ET AL: "Rapid and sensitive detection of Chlamydia trachomatis sexually transmitted infections in resource-constrained settings in Thailand at the point-of-care", vol. 12, no. 12, 20 December 2018 (2018-12-20), US, pages e0006900, XP093192040, ISSN: 1935-2735, Retrieved from the Internet <URL:https://journals.plos.org/plosntds/article/file?id=10.1371/journal.pntd.0006900&type=printable> DOI: 10.1371/journal.pntd.0006900
- I. CHOOPARA ET AL: "Rapid and visual Chlamydia trachomatis detection using loop-mediated isothermal amplification and hydroxynaphthol blue", LETTERS IN APPLIED MICROBIOLOGY, vol. 64, no. 1, 1 January 2016 (2016-01-01), GB, pages 51 - 56, XP055483564, ISSN: 0266-8254, DOI: 10.1111/lam.12675
- BIRKELUND S ET AL: "The 75-kilodalton cytoplasmic Chlamydia trachomatis L2 polypeptide is a DnaK-like protein", INFECTION AND IMMUNITY, vol. 58, no. 7, 1 July 1990 (1990-07-01), US, pages 2098 - 2104, XP093192158, ISSN: 0019-9567, DOI: 10.1128/iai.58.7.2098-2104.1990
- SOMBOONNA NARAPORN, CHOOPARA ILADA, ARUNRUT NARONG, SUKHONPAN KANCHAPAN, SAYASATHID JARUN, DEAN DEBORAH, KIATPATHOMCHAI WANSIKA: "Rapid and sensitive detection of Chlamydia trachomatis sexually transmitted infections in resource-constrained settings in Thailand at the point-of-care", PLOS NEGL TROP DIS, 1 January 2018 (2018-01-01), XP055933570, Retrieved from the Internet <URL:https://journals.plos.org/plosntds/article/file?id=10.1371/journal.pntd.0006900&type=printable> [retrieved on 20220621]
- TRUDI COLLET; TOM MACNAUGHTON; TERENCE WALSH; JOSEPH DEBATTISTA; PETER TIMMS;: "Identification of novel markers for uncomplicated lower genital tract infections and upper genital tract pathology due to", INTERNATIONAL JOURNAL OF INFECTIOUS DISEASES, INTERNATIONAL SOCIETY FOR INFECTIOUS DISEASES, HAMILTON, CA, vol. 15, no. 4, 19 December 2010 (2010-12-19), CA , pages e257 - e266, XP028165419, ISSN: 1201-9712, DOI: 10.1016/j.ijid.2010.12.005

## Description

The present invention relates to a set of primers for the detection of *Chlamydia trachomatis* (ChT) bacteria, a method for the detection of *Chlamydia trachomatis* using the set of primers, and the use of the set of primers for the detection of Chlamydia trachomatis bacteria. The invention is applicable in medical diagnostics.

*Chlamydia trachomatis* is an atypical bacteria characterized by its lack of a cell wall. It causes atypical, intracellular infections of the genitourinary tract, in which the basic replication unit is the Reticulate Body (RB), which then transforms into the Elementary Body (EB) capable of infecting other host cells or infecting another individual.

*Chlamydia trachomatis* is one of the most frequently diagnosed infectious agents among sexually transmitted diseases (STDs). *Chlamydia trachomatis* is responsible for 60% of sexually transmitted bacterial infections¹ among sexually active people aged 19-49, in particular serovars I, II and III. Beyond causing urogenital infections, *Chlamydia trachomatis* (in particular serovars A, B, Ba, C, D, Da, E, F, G, H, I, Ia, J, Ja,) is also an etiological factor of trachoma, chronic conjunctivitis, affecting an average of 40 million people each year, mainly in countries with low economic rates².
¹ CDC. Summary of notifiable diseases and conditions -- united states, 2015. MMWR 64, 1-143 (2017).
² Bhosai, S. J., Bailey, R. L., Gaynor, B. D. & Lietman, T. M. Trachoma: an updateon prevention, diagnosis, and treatment.Curr. Opin. Ophthalmo1.23, 288-295(2012).

Due to the lack of a cell wall, these bacteria are resistant to β-lactam antibiotics - the most frequently used group of antibiotics in the first line of treatment, which is one of

the main difficulties in treating *Chlamydia trachomatis* infections. Another difficulty is the diagnostic process of *Chlamydia trachomatis* infections. Atypical bacteria require specific growth conditions (cell lines or specialized media), which makes it difficult to detect the bacteria in biological material using classical microbiological methods. In turn, untreated infection or delayed treatment can lead to numerous complications. The most common complications in women are pelvic inflammation, ectopic pregnancy or infertility caused by tubal dysfunction, while untreated *Chlamydia trachomatis* infection in men leads to inflammation of the epididymis and rectum³.
³ Rekart ML, Gilbert M, Meza R, Kim PH, Chang M, Money DM, et al. Chlamydia public health programs and the epidemiology of pelvic inflammatory disease and ectopic pregnancy. J Infect Dis 2013;207:30-8.

Laboratory diagnostics of *Chlamydia trachomatis* bacteria, due to the intracellular nature of bacterial reproduction and dependence on host ATP, is complicated and traditional microbiological methods are not effective. Cell culture of bacteria, although highly specific, is laborious, expensive, requires expensive equipment, and the results are obtained only after about 3 days. Immunological methods based on the detection of bacterial antigens or antibodies (IgG, IgM or IgA class) produced by the host (e.g. ELISA, enzyme-linked immunosorbent assay) are characterized by low sensitivity and specificity, and may also indicate a past disease, not referring to the current condition of the patient.

The methods characterized by the greatest specificity and sensitivity are those involving the detection of *Chlamydia trachomatis* nucleic acid in biological material (the so-called NAAT methods - Nucleic Acid Amplification Tests), i.e. in the urine or urethral swab in men and vaginal or urethral swab in women. The most commonly used tests in NAAT technology

are Real-Time PCR-based assays. Many different tests using the Real-Time PCR technique are available on the market, but despite the strong competition, these methods are still relatively expensive. Moreover, they require highly specialized personnel, expensive devices and require the isolation of genetic material from the patient's sample. Moreover, since cyclic heating and cooling of the reagents is necessary, this method is relatively long, and the devices used use relatively much energy to carry out this process.

Isothermal methods - including the LAMP (Loop-mediated isothermal amplification) method, are methods that allow to speed up the diagnostic process, reduce the cost of energy needed to perform the analysis and allow the determination to be performed with no need to isolate DNA/RNA of the detected pathogen. Moreover, according to the literature data, these methods are characterized by higher sensitivity and specificity than the aforementioned Real-Time PCR technique, they are also much faster. The isothermal course does not require specialized equipment.

Due to the low hardware requirements, isothermal methods are an ideal diagnostic solution for primary care units (POCT - point-of-care testing), where the test can be performed in the practice of a general practitioner or medical specialist (gynecologist, urologist) at the first contact of a patient with the doctor. This solution allows for a quick diagnostic test (in no more than 15 minutes), which allows for selection of a targeted therapy during the very first visit. This is especially important with atypical bacteria such as *Chlamydia trachomatis.* On the other hand, the use of lyophilized reagents allows the tests to be stored at room temperature, without the need to freeze the diagnostic tests.

The use of primers in the LAMP method for the diagnosis of *Chlamydia trachomatis* is known from the patent applications published so far: JP2019507606A; US20190111423A1; CN110177887A; KR101958048B1; CN107099618A; US20150322493A1. The LAMP method is disclosed, for example, in WO0028082, WO0224902, WO 2017/103269 A1 . The mentioned patent applications in most cases do not describe the sensitivity and detection limit of *Chlamydia trachomatis* bacteria. The detection method in some of the above-mentioned patent applications does not allow for quantitative measurement, and the detection is of the end-point type, using agarose gel or other markers based on the color change of the reaction mixture upon a positive result of the amplification reaction (Hydroxy-Naphthol-Blue, Calceine). Some patent applications are realized in the Real-Time technology, which enables quantitative measurement, but the detection method is based on Molecular Beacon probes labeled with double fluorescent dyes, which increases the costs of the analysis, or with radioisotopes (RI) that require a specific workflow with such material. Moreover, most of the kits developed and described above are not applicable in POCT diagnostics, and their main application is in laboratories.

Therefore, there is still a need to provide a diagnostic method using appropriately refined sets of primers used for the diagnosis of *Chlamydia trachomatis* with the LAMP method, intended for use in point-of-care testing, which allows the detection of bacteria with a very low detection limit (≥ 5 copies/reaction) in a short time (≤ 15 min). Unexpectedly, the above problem was solved by the present invention.

The invention is defined in the appended claims.

The first subject of the invention is a set of primers for amplifying the nucleotide sequence of the *Chlamydia trachomatis dnaK* gene, characterized in that it contains a set of internal primers with the following nucleotide sequences a) and b), as well as a set of external primers containing the following nucleotide sequences c) and d) specific for the selected fragment of the *Chlamydia trachomatis dnaK* gene:
a) 5' GCGAGTTAGAGTTAAAGCCAAATGT 3' (nucleic sequence SEQ ID NO: 3 or its reverse and complementary sequence, linked or not linked from the 3' end by a TTTT bridge to the sequence 5' TCTACTGAAATCAATCAGCCATT 3'- (nucleic sequence SEQ ID NO: 4 or its reverse and complementary sequence;
b) 5' TCGAACACCTAGCTTCCTCTCT 3'- (nucleic sequence SEQ ID NO: 5 or its reverse and complementary sequence, linked or not linked from the 3' end by a TTTT bridge to the sequence 5' GAAGCGGACAATTTAGCAT 3'- (nucleic sequence SEQ ID NO: 6 or its reverse and complementary sequence;
c) 5' AATAGAATTGTCTGGTGTATCG 3' nucleic sequence SEQ ID NO: 1 or its reverse and complementary sequence, and
d) 5' CTAGAAGAACATCATCAATGTCA 3' nucleic sequence SEQ ID NO: 2 or its reverse and complementary sequence wherein the primer set comprises a set of loop primer sequences comprising nucleic sequences contained in or complementary to the *Chlamydia trachomatis dnaK* and having the nucleic acid sequences SEQ ID NO: 7 - 5' GGTCCATTAGCGTCGATAGTGATG 3' and SEQ ID NO: 8: 5' CCAAACAACCTTGTGCTCAGGCT 3' or sequences reverse and complementary to them.

The second subject of the invention is a method for detecting *Chlamydia trachomatis* bacteria, characterized in that a selected region of the nucleic sequence of the *Chlamydia trachomatis* genome (*dnaK* gene fragment) is amplified using a primer set as defined in the first subject of the invention, the amplification method being the LAMP method.

In a preferred embodiment, the amplification is carried out with a temperature profile of: 64°C, 40 min.

In a further preferred embodiment of the invention, the end-point reaction is carried out with a temperature profile of 80°C, 5 min.

The third subject of the invention is a method for the detection of an infection caused by *Chlamydia trachomatis* bacterium, characterized in that it comprises a detection method as defined in the second subject of the invention.

The fourth subject of the invention is a kit for the detection of an infection caused by *Chlamydia trachomatis,* characterized in that it comprises a set of primers as defined in the first subject of the invention.

In a preferred embodiment of the invention, the infection detection kit comprises 5.0 µl of WarmStart LAMP Master Mix.

In a further preferred embodiment of the invention, individual amplification primers as defined in the first subject of the invention, the primers having the following concentrations: 0.13 µM F3, 0.13 µM B3, 1.06 µM FIP, 1.06 µM BIP, 0.27 µM LoopF, 0.27 µM LoopB; D-(+)-Trehalose dihydrate - 6%; fluorescent marker interacting with double-stranded DNA - EvaGreen ≤1X or Fluorescent Dye in the amount of ≤0.5 µl or GreenFluorescent Dye in the amount of ≤1 µl or Syto-13 ≤16 µM or SYTO-82 ≤16 µM or another fluorescent dye interacting with double-stranded DNA at a concentration that does not inhibit the amplification reaction.

The advantage of the primer sets of the invention for the detection of *Chlamydia trachomatis,* as well as the method for the detection of *Chlamydia trachomatis* infection and the method of detecting the amplification products is the possibility of using them in medical diagnostics at the point of care (POCT) in the target application with a portable genetic analyzer. The lyophilization of the reaction mixtures of the invention allows the diagnostic kits to be stored at room temperature without reducing the diagnostic parameters of the tests. In turn, the use of a fluorescent dye to detect the amplification product increases the sensitivity of the method, allows to lower the detection limit (down to 5 copies/reaction), and also enables the quantitative measurement of bacteria in the test sample.

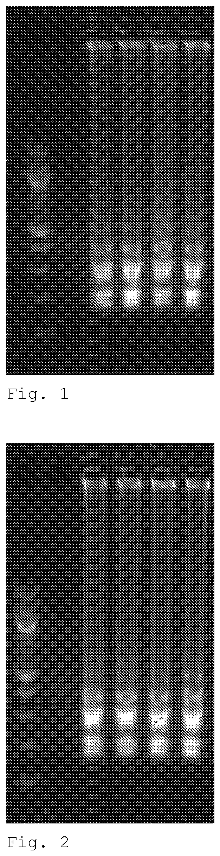

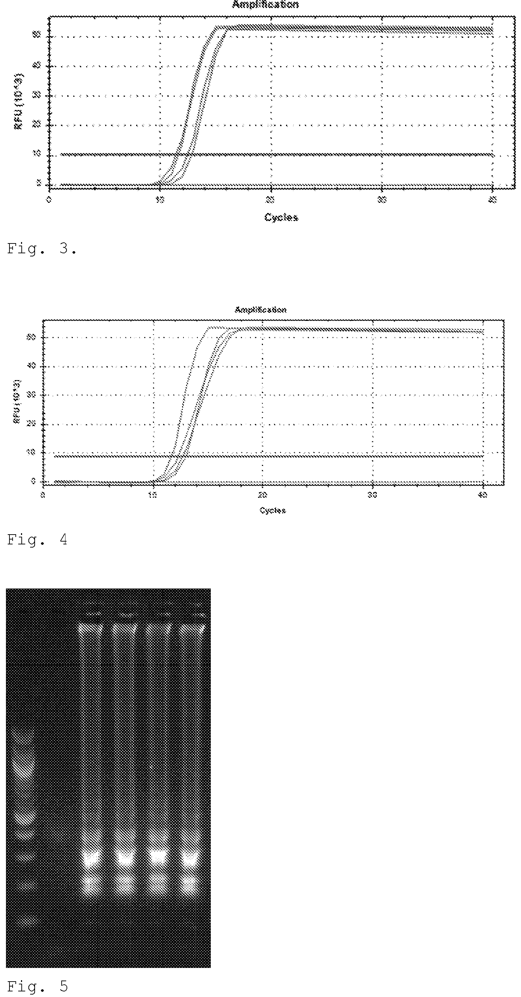

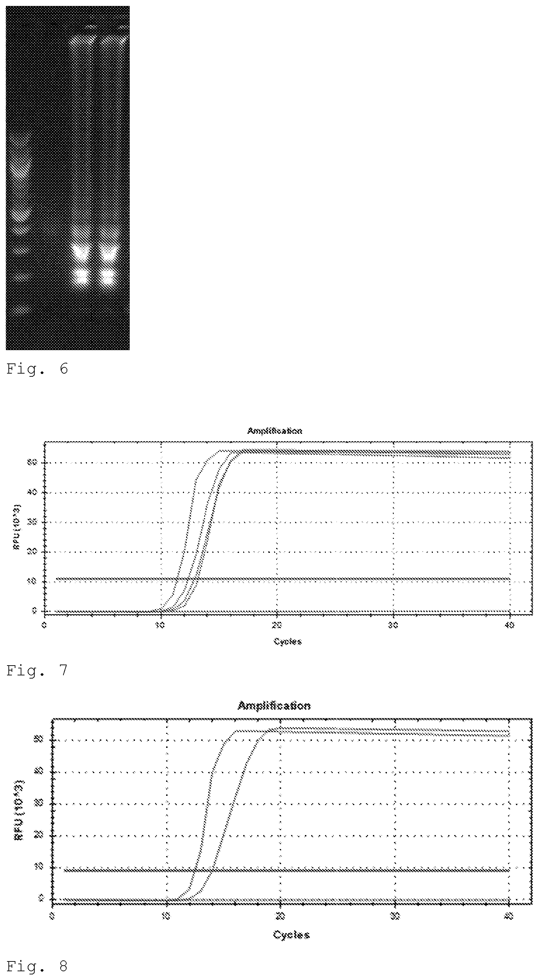

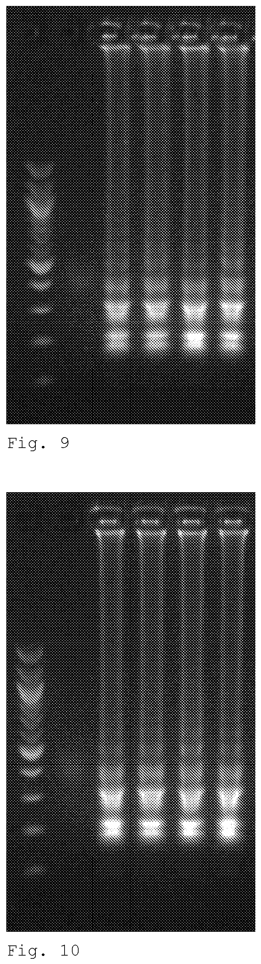

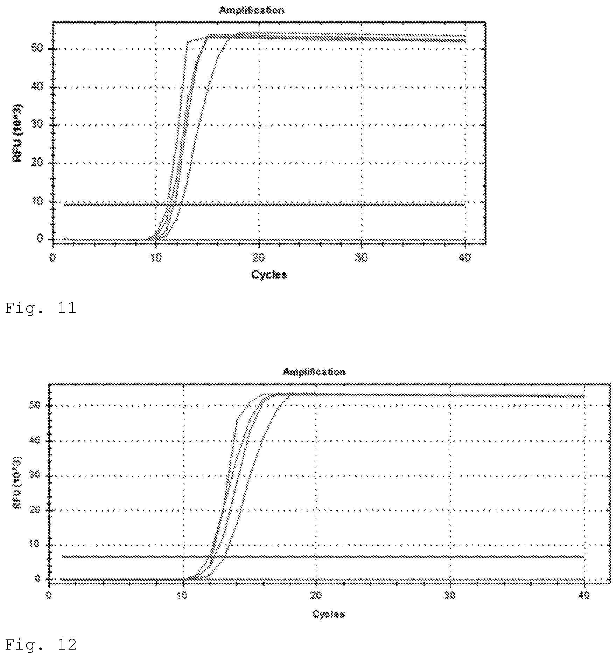

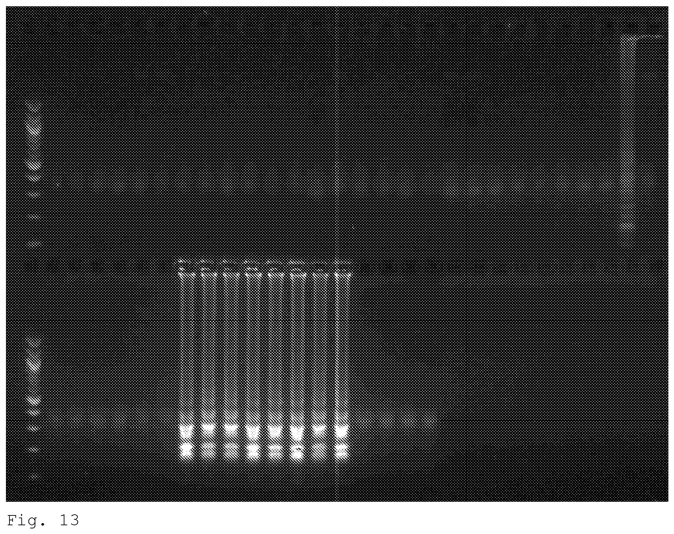

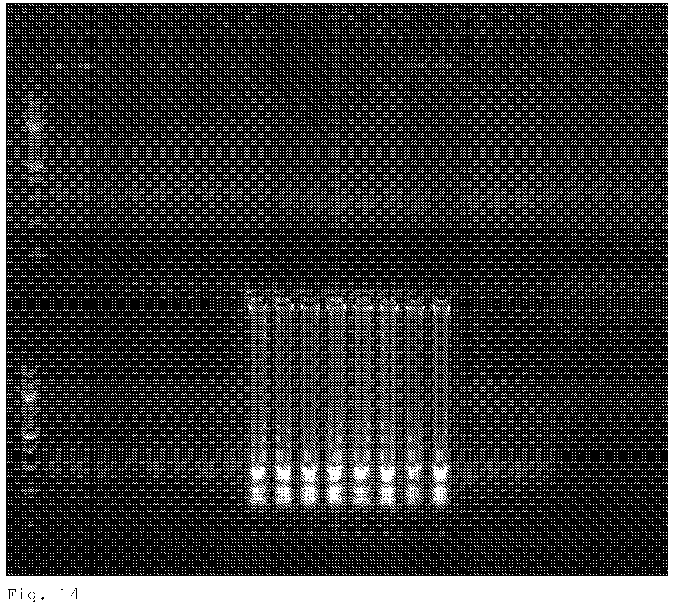

Exemplary implementations of the invention are presented in the figures, in which Figs. 1 and 2 show the sensitivity characteristics of the method, where a specific signal was obtained with the template: Genomic DNA from *Chlamydia trachomatis* LGV Serovar I (ATCC^{®} VR-901BD^{™}) over the range of 100-5 copies/µl, but there was no product in NTC; Fig. 1: lane 1: mass marker (Quick-Load^{®} Purple 100 bp DNA Ladder, NewEngland Biolabs); lane 2: NTC; lane 3: 10 copies of ChT; lane 4: 20 copies of ChT; lane 5: 50 copies of ChT; lane 6: 100 copies of ChT; Fig. 2: lane 1: mass marker (Quick-Load^{®} Purple 100 bp DNA Ladder, NewEngland Biolabs); lane 2: NTC; lane 3: 5 copies of ChT; lane 4: 10 copies of ChT; lane 5: 20 copies of ChT; lane 6: 50 copies of ChT, Fig. 3 shows the sensitivity of the method of the invention measured by assaying a serial dilution of the Genomic DNA standard from *Chlamydia trachomatis* LGV Serovar I (ATCC^{®} VR-901BD^{™}) over the range of 100-10 copies/µl of the DNA standard, where the product amplification was measured in real time. The results of real-time *Chlamydia trachomatis* detection are shown in Table 1, giving the minimum time needed to detect the fluorescence signal, Fig. 4 shows the sensitivity of the method of the invention, measured by assaying a serial dilution of the Genomic DNA standard from *Chlamydia trachomatis* LGV Serovar I (ATCC^{®} VR-901BD^{™}) over the range of 50-5 copies/µl of the DNA standard, where the product amplification was measured in real time. The results of real-time *Chlamydia trachomatis* detection are shown in Table 2, giving the minimum time needed to detect the fluorescence signal, Figs. 5 and 6 show the sensitivity characteristics of the method, where a specific signal was obtained with the template: Genomic DNA from *Chlamydia trachomatis* LGV Serovar II (ATCC^{®} VR -902BD^{™}) over the range of 100-5 copies/µl, but there was no product in NTC; Fig. 5: lane 1: mass marker (Quick-Load^{®} Purple 100 bp DNA Ladder, NewEngland Biolabs); lane 2: NTC; lane 3: 10 copies of ChT; lane 4: 20 copies of ChT; lane 5: 50 copies of ChT; lane 6: ChT 100 copies; Fig. 6: lane 1: mass marker (Quick-Load^{®} Purple 100 bp DNA Ladder, NewEngland Biolabs); lane 2: NTC; lane 3: 5 copies of ChT; lane 4: 25 copies of ChT, Fig. 7 shows the sensitivity of the method of the invention measured by assaying a serial dilution of the Genomic DNA standard from *Chlamydia trachomatis* LGV Serovar II (ATCC^{®} VR-902BD^{™}) over the range of 100-10 copies/µl of the DNA standard, where the product amplification was measured in real time. The results of realtime *Chlamydia trachomatis* detection are shown in Table 3, giving the minimum time needed to detect the fluorescence signal, Fig. 8 shows the sensitivity of the method of the invention measured by assaying a serial dilution of the DNA standard from *Chlamydia trachomatis* LGV Serovar II (ATCC^{®} VR-902BD^{™}) over the range of 25-5 copies/µl of the DNA standard, where the product amplification was measured in real time. The results of realtime *Chlamydia trachomatis* detection are shown in Table 4, giving the minimum time needed to detect the fluorescence signal, Figs. 9 and 10 show the sensitivity characteristics of the method, where a specific signal was obtained with the template: Genomic DNA from *Chlamydia trachomatis* LGV Serovar III (ATCC^{®} VR-903D^{™}) over the range of 100-5 copies/µl, but there was no product in NTC; Fig. 1: lane 1: mass marker (Quick-Load^{®} Purple 100 bp DNA Ladder, NewEngland Biolabs); lane 2: NTC; lane 3: 10 copies of ChT; lane 4: 20 copies of ChT; lane 5: 50 copies of ChT; lane 6: ChT 100 copies, Figure 10: lane 1: mass marker (Quick-Load^{®} Purple 100 bp DNA Ladder, NewEngland Biolabs); lane 2: NTC; lane 3: 5 copies of ChT; lane 4: 10 copies of ChT; lane 5: 20 copies of ChT; lane 6: 50 copies of ChT, Fig. 11 shows the sensitivity of the method of the invention measured by assaying a serial dilution of the Genomic DNA standard from *Chlamydia trachomatis* LGV Serovar III (ATCC^{®} VR-903D^{™}) over the range of 100-10 copies/µl of the DNA standard, where the product amplification was measured in real time. The results of the real-time *Chlamydia trachomatis* detection are shown in Table 5, giving the minimum time needed to detect a fluorescence signal, Fig. 12 shows the sensitivity of the method of the invention measured by assaying a serial dilution of the Genomic DNA from *Chlamydia trachomatis* LGV Serovar III standard (ATCC^{®} VR-903D^{™}) over the range of 50-5 copies/µl of DNA standard, where the product amplification was measured in real time. The results of the real-time *Chlamydia trachomatis* detection are shown in Table 6, giving the minimum time needed to detect the fluorescence signal, Figs. 13 and 14 show the specificity of the method of the invention with standard matrices of a number of pathogens potentially present in the tested biological material as natural physiological flora, those which may result from co-infections or those which share similar genomic sequences. Fig 13: lane 1: mass marker (Quick-Load^{®} Purple 100 bp DNA Ladder, NewEngland Biolabs); lanes 2 and 3: *Mycoplasma genitalium;* lanes 4 and 5: *Klebsiella pneumoniae;* lanes 6 and 7: *Bordetella pertussis;* lanes 8 and 9: *Neisseria gonorrhoeae;* lanes 10 and 11: methicillin-resistant *Staphylococcus aureus* (MRSA); lanes 12 and 13: *Lactobacillus jensenii;* lanes 14 and 15: *Enterococcus faecalis;* lanes 16 and 17: *Enterococcus faecium;* lanes 18 and 19: *Staphylococcus epidermidis;* lanes 20 and 21: *Pseudomonas aeruginosa;* lanes 22 and 23: *Streptococcus agalactiae;* lanes 24 and 25: methicillinsensitive *Staphylococcus aureus* (MSSA); lanes 26 and 27: *Mycoplasma hominis;* lanes 28 and 29: *Haemophilus ducreyi;* lane 30: mass marker (Quick-Load^{®} Purple 100 bp DNA Ladder, NewEngland Biolabs); lanes 31 and 32: CMV; lanes 33 and 34: *Lactobacillus gasseri;* lanes 35 and 36: human DNA; lanes 37 and 38: *Chlamydia trachomatis* Serovar D; lanes 39 and 40: *Chlamydia trachomatis* Serovar G; lanes 41 and 42: *Chlamydia trachomatis* Serovar J; lanes 43 and 44: *Chlamydia trachomatis* Serovar H; lanes 45, 46, 47, 48: NTC and Fig. 14: lane 1: mass marker (Quick-Load^{®} Purple 100 bp DNA Ladder, NewEngland Biolabs); lanes 2 and 3: *Listeria monocytogenes;* lanes 4 and 5: *Bacteroides fragilis;* lanes 6 and *7: Escherichia coli;* lanes 8 and 9: *Mobiluncus mulieris;* lanes 10 and 11: *Trichomonas vaginalis;* lanes 12 and 13: *Gardnerella vaginalis;* lanes 14 and 15: *Ureoplasma urealyticum;* lanes 16 and *17: Campylobacter jejuni;* lanes 18 and 19: HBV; lanes 20 and 21: HHV-1; lanes 22 and 23: HPV 16; lanes 24 and 25: HPV 18; lane 26: mass marker (Quick-Load^{®} Purple 100 bp DNA Ladder, NewEngland Biolabs); lanes 27 and 28: HSV 2; lanes 29 and 30: *Treponema pallidum;* lanes 31 and 32: *Candida albicans;* lanes 33 and 34: *Chlamydiophila pneumoniae;* lanes 35 and 36: *Chlamydia trachomatis* Serovar E; lanes 37 and 38: *Chlamydia trachomatis* Serovar I; lanes 39 and 40: *Chlamydia trachomatis* Serovar II; lanes 41 and 42: *Chlamydia trachomatis* Serovar III; lanes 43, 44, 45, 46: NTC.

### Example 1. Primer sequences

The sequences of specific oligonucleotides used for the detection of *Chlamydia trachomatis* genetic material using LAMP technology are presented and characterized below.
1. The ChT *dnaKF3* oligonucleotide sequence: 5' AATAGAATTGTCTGGTGTATCG 3' is identical to the *Chlamydia trachomatis dnaK* gene (5'-3' strand), which is 3' adjacent to the F2 primer.
2. The ChT *dnaKB3* oligonucleotide sequence: 5' CTAGAAGAACATCATCAATGTCA 3' is a complementary fragment of the *Chlamydia trachomatis dnaK* gene (5'-3' strand) 164 nucleotides away from the 3' end of the oligonucleotide 1.
3. The ChT *dnaKF2* oligonucleotide sequence: 5' TCTACTGAAATCAATCAGCCATT 3' is a sequence identical to the *Chlamydia trachomatis dnaK* gene (5'-3' strand) immediately adjacent to the 3' end of the oligonucleotide 1.
4. The ChT *dnaKB2* oligonucleotide sequence: 5' GAAGCGGACAATTTAGCAT 3' is a complementary fragment of the *Chlamydia trachomatis dnaK* gene (5'-3' strand) 145 nucleotides away from the 3' end of the oligonucleotide 1.
5. The ChT *dnaKF*1c oligonucleotide sequence: 5' GCGAGTTAGTTAAAGCCAAATGT 3' is a complementary fragment of the *Chlamydia trachomatis dnaK* gene (5'-3' strand) 50 nucleotides away from the 3' end of the oligonucleotide 1.
6. The ChT *dnaKB*1c oligonucleotide sequence: 5' TCGAACACCTAGCTTCCTCTCT 3' is identical to the *Chlamydia trachomatis dnaK* gene (5'-3' strand) 82 nucleotides away from the 3' end of the oligonucleotide 1.
7. The ChT dnaKLoopF oligonucleotide sequence: 5' GGTCCATTAGCGTCGATAGTGATG 3'.
8. The ChT dnaKLoopB oligonucleotide sequence: 5' CCAAACAACCTTGTGCTCAGGCT 3'.

The sequences of the F1c and F2 oligonucleotides have preferably been linked by a TTTT bridge and used as FIP. The sequences of the B1c and B2 oligonucleotides were preferably linked by a TTTT bridge and used as BIP.

### Example 3

Method of amplifying the *Chlamydia trachomatis dnaK* gene using the oligonucleotides characterized in Example 1 with LAMP technology and the following composition of the reaction mixture:
5.0 µl WarmStart LAMP 2X Master Mix
0.13 µM F3
0.13 µM B3
1.06 µM FIP
1.06 µM BIP
0.27 µM LoopF
0.27 µM LoopB
D-(+)-Trehalose dihydrate - 6%

Fluorescent marker interacting with double-stranded DNA - EvaGreen ≤1X or Fluorescent dye 50X (New England Biolabs) in the amount of 0.5 µl or GreenFluorescent Dye (Lucigen) in the amount of ≤1 µl or Syto-13 ≤16 µM or SYTO-82 ≤16 µM or another fluorescent dye that interacts with double-stranded DNA at a concentration that does not inhibit the amplification reaction.
DNA template ≥5 copies/reaction
Total reaction volume adjusted to 10 µl with DNase and RNase free water.

### Example 4

Method of amplifying the *Chlamydia trachomatis dnaK* gene using the oligonucleotides characterized in Example 1 and Example 3 with LAMP technology and the composition of the reaction mixture characterized in Example 3 with the following temperature profile:
1) 64°C, 40 min
2) preferably for end-point reactions 80°C, 5 min.

### Example 5

Method of amplification and detection of the *Chlamydia trachomatis dnaK* gene using the oligonucleotides characterized in Example 1 and Example 3 with LAMP technology and the composition of the reaction mixture characterized in Example 3 with the temperature profile characterized in Example 4 and the detection method described below.

A fluorescent dye is used, capable of interacting with double-stranded DNA, added to the reaction mixture in an amount of 0.5 µl EvaGreen 20X; 0.5 µL or a concentration of ≤1X; ≤16 µM respectively for GreenFluorescent Dye (Lucigen); SYTO-13 and SYTO-82 before starting the reaction, real-time and/or end-point measurement. Excitation wavelength in the range similar to the FAM dye - 490-500 nm (optimally 494 nm) for EvaGreen; Fluorescent dye 50X (New England Biolabs), GreenFluorescent Dye (Lucigen); SYTO-13 dyes and 535 nm (optimally 541 nm) for SYTO-82 dye; emission wavelength in the range 509-530 nm (optimally 518 nm) for EvaGreen; GreenFluorescent Dye (Lucigen); SYTO-13 dyes and 556 nm (optimally 560 nm) for SYTO-82 dye, the method of detection, change registration time starting from 11 minutes from the start of the reaction for *Chlamydia trachomatis* and the negative control.

### Example 6

The method of preparation and lyophilization of reagents for detecting the amplification and detection of the *Chlamydia trachomatis dnaK* gene using the oligonucleotides characterized in Example 1 and Example 3 with LAMP technology and the composition of the reaction mixture characterized in Example 3 with the temperature profile characterized in Example 4 and the detection method described in Example 5.

### Example 7. Description of the lyophilization process

The reaction components were mixed according to the composition described in Example 3, except the template DNA, to a total volume of 10 µl. The mixture was transferred to 0.2 ml tubes and subjected to the lyophilization process according to the parameters below.

The mixture placed in the test tubes was pre-cooled to -80°C for 2 hours. Then the lyophilization process was carried out at the temperature of -80°C for 3 hours under the pressure of 5⁻² mBar.

### Example 8. Sensitivity of the method

The sensitivity was determined by assaying serial dilutions of the standards: Genomic DNA from *Chlamydia trachomatis* LGV Serovar I (ATCC^{®} VR-901BD^{™}), Genomic DNA from *Chlamydia trachomatis* LGV Serovar II (ATCC^{®} VR-902BD^{™}), Genomic DNA from *Chlamydia trachomatis* LGV Serovar III (ATCC^{®} VR-903D^{™}) with a minimum amount of 5 copies of bacteria per reaction mixture, where the product amplification was measured in real time - Figures 3, 4, 7, 8, 11, 12 (RealTime-LAMP for serial dilutions). The time required to detect the emitted fluorescence for individual samples is shown in Tables 1, 2, 3, 4, 5, 6.

The characterized primers allow for the detection of *Chlamydia trachomatis* bacteria by detecting the *dnaK* gene fragment at a minimum number of 5 copies/reaction mixture.

**Table 1. Time required to detect fluorescence for each dilution of the Genomic DNA from Chlamydia trachomatis LGV Serovar I (ATCC^{®} VR-901BD^{™}) standard.**

| Sample | Time to exceed the baseline fluorescence [min] |
|---|---|
| ChT NTC | Indefinite |
| ChT 10 copies | 12.82 |
| ChT 20 copies | 12.45 |
| ChT 50 copies | 11.63 |
| ChT 100 copies | 11.42 |

**Table 2. Time required to detect fluorescence for each dilution of the Genomic DNA from Chlamydia trachomatis LGV Serovar I (ATCC^{®} VR-901BD^{™}) standard.**

| Sample | Time to exceed the baseline fluorescence [min] |
|---|---|
| ChT NTC | Indefinite |
| ChT 5 copies | 12.96 |
| ChT 10 copies | 12.64 |
| ChT 20 copies | 12.24 |
| ChT 50 copies | 11.61 |

**Table 3. Time required to detect fluorescence for each dilution of the Genomic DNA from Chlamydia trachomatis LGV Serovar II (ATCC^{®} VR-902BD^{™}) standard.**

| Sample | Time to exceed the baseline fluorescence [min] |
|---|---|
| ChT NTC | Indefinite |
| ChT 10 copies | 13.14 |
| ChT 20 copies | 12.86 |
| ChT 50 copies | 12.31 |
| ChT 100 copies | 11.36 |

**Table 4. Time required to detect fluorescence for each dilution of the Genomic DNA from Chlamydia trachomatis LGV Serovar II (ATCC^{®} VR-902BD^{™}) standard.**

| Sample | Time to exceed the baseline fluorescence [min] |
|---|---|
| ChT NTC | Indefinite |
| ChT 5 copies | 13.99 |
| ChT 25 copies | 12.48 |

**Table 5. Time required to detect fluorescence for each dilution of the Genomic DNA from Chlamydia trachomatis LGV Serovar III (ATCC^{®} VR-903D^{™}) standard.**

| Sample | Time to exceed the baseline fluorescence [min] |
|---|---|
| ChT NTC | Indefinite |
| ChT 10 copies | 12.38 |
| ChT 20 copies | 11.68 |
| ChT 50 copies | 11.36 |
| ChT 100 copies | 11.08 |

**Table 6. Time required to detect fluorescence for each dilution of the Genomic DNA from Chlamydia trachomatis LGV Serovar III (ATCC^{®} VR-903D^{™}) standard.**

| Sample | Time to exceed the baseline fluorescence [min] |
|---|---|
| ChT NTC | Indefinite |
| ChT 5 copies | 13.06 |
| ChT 10 copies | 12.29 |
| ChT 20 copies | 12.13 |
| ChT 50 copies | 11.94 |

The superiority of the amplification method and the oligonucleotides described in this specification over the tests based on the RealTime-LAMP technology is due to the much higher sensitivity, which is shown in Figures 1, 2, 5, 6, 9, 10 and the reduction of the analysis time shown in Figures 3, 4, 7, 8, 11, 12 and Tables 1, 2, 3, 4, 5, 6.

## Claims

1. A set of primers for amplifying the nucleotide sequence of the *Chlamydia trachomatis dnaK* gene, **characterized in that** it contains a set of internal primers with the following nucleotide sequences a) and b), as well as a set of external primers containing the following nucleotide sequences c) and and d):
a) 5' GCGAGTTAGAGTTAAAGCCAAATGT 3'- (nucleic sequence SEQ ID NO: 3 or its reverse and complementary sequence), linked or not linked from the 3' end by a TTTT bridge to the sequence 5' TCTACTGAAATCAATCAGCCATT 3'- (nucleic sequence or SEQ ID NO: 4 or sequence reverse and complementary sequence)
b) 5' TCGAACACCTAGCTTCCTCTCT 3'- (nucleic sequence SEQ ID NO: 5 or its reverse and complementary sequence), linked or not linked from the 3' end by a TTTT bridge to the sequence 5' GAAGCGGACAATTTAGCAT 3'- (nucleic sequence SEQ ID NO: 6 or its reverse and complementary sequence)
c) 5' AATAGAATTGTCTGGTGTATCG 3' nucleic sequence SEQ ID NO: 1 or its reverse and complementary sequence, and
d) 5' CTAGAAGAACATCATCAATGTCA 3' nucleic sequence SEQ ID NO: 2 or its reverse and complementary sequence, wherein
it contains a set of loop primer sequences containing nucleotide sequences contained in or complementary to the *Chlamydia trachomatis dnaK* gene SEQ ID NO: 7 - 5' GGTCCATTAGCGTCGATAGTGATG 3' and SEQ ID NO: 8: 5' CCAAACAACCTTGTGCTCAGGCT 3' or sequences reverse and complementary to them.

2. A method of detecting *Chlamydia trachomatis* bacteria, **characterized in that** a selected region of the nucleic sequence of the bacterial genome is amplified using the set of primers as defined in claim 1, the amplification method being the LAMP method.

3. The method of detecting bacteria of claim 2, **characterized in that** the amplification is carried out with a temperature profile of: 64°C, 40 min.

4. The method of claim 3, **characterized in that** for the end-point detection reaction is carried out with a temperature profile of 80°C, 5 min.

5. A method for the detection of a *Chlamydia trachomatis* bacterial infection, **characterized in that** it comprises the detection method of claim 2.

6. A kit for the detection of *Chlamydia trachomatis* infection, **characterized in that** it comprises a set of primers as defined in claim 1.

7. The kit of claim 6,
**characterized in that** it contains the amplification primers as defined in claim 1 and in claim 2, wherein the primers have the following concentrations: 0.13 µM F3, 0.13 µM B3, 1.06 µM FIP, 1.06 µM BIP, 0.27 µM LoopF, 0.27 µM LoopB; D-(+)-Trehalose dihydrate - 6%; fluorescent marker interacting with double-stranded DNA - EvaGreen ≤1X or Fluorescent Dye in the amount of ≤0.5 µl or GreenFluorescent Dye in the amount of ≤1 µl or Syto-13 ≤16 µM or SYTO-82 ≤16 µM or another fluorescent dye interacting with double-stranded DNA at a concentration that does not inhibit the amplification reaction.

## Patentansprüche

1. Primersatz zur Amplifizierung der Nukleotidsequenz des dnak-Gens von *Chlamydia trachomatis,* **dadurch gekennzeichnet, dass** er einen Satz von internen Primern mit den folgenden Nukleotidsequenzen a) und b) sowie einen Satz von externen Primern mit den folgenden Nukleotidsequenzen c) und d) enthält:
a) 5'-GCGAGTTAGAGTTAAAGCCAAATGT-3' - (Nukleinsequenz SEQ ID NO: 3 oder ihre umgekehrte und komplementäre Sequenz), die vom 3'-Ende durch eine TTTT-Brücke mit der Sequenz 5'-TCTACTGAAATCAATCAGCCATT-3' (Nukleinsequenz oder SEQ ID NO: 4 oder umgekehrte und komplementäre Sequenz) verbunden ist oder nicht
b) 5'-TCGAACACCTAGCTTCCTCTCT-3' - (Nukleinsequenz SEQ ID NO: 5 oder ihre umgekehrte und komplementäre Sequenz), die vom 3'-Ende durch eine TTTT-Brücke mit der Sequenz 5'-GAAGCGGACAATTTAGCAT-3' - (Nukleinsequenz SEQ ID NO: 6 oder ihre umgekehrte und komplementäre Sequenz) verbunden ist oder nicht
c) 5'-AATAGAATTGTCTGGTGTATCG-3' Nukleinsequenz, SEQ ID NO: 1 oder ihre umgekehrte und komplementäre Sequenz, und
d) 5'-CTAGAAGAACATCATCAATGTCA-3' Nukleinsequenz, SEQ ID NO: 2 oder ihre umgekehrte und komplementäre Sequenz, wobei er einen Satz von Schleifen-Primersequenzen enthält, die Nukleotidsequenzen enthalten, die im dnak-Gen von *Chlamydia trachomatis* SEQ ID NO: 7 5'-GGTCCATTAGCGTCGATAGTGATG-3' und SEQ ID NO: 8 5'-CCAAACAACCTTGTGCTCAGGCT-3' oder dazu umgekehrten und komplementäre Sequenzen enthalten oder komplementär dazu sind.

2. Verfahren zum Nachweis von *Chlamydia* trachomatis-Bakterien, **dadurch gekennzeichnet, dass** ein ausgewählter Bereich der Nukleussequenz des bakteriellen Genoms unter Verwendung des in Anspruch 1 definierten Satzes von Primern amplifiziert wird, wobei das Amplifikationsverfahren das LAMP-Verfahren ist.

3. Verfahren zum Nachweisen von Bakterien nach Anspruch 2, **dadurch gekennzeichnet, dass** die Amplifikation mit einem Temperaturprofil durchgeführt wird von: 64 °C, 40 min.

4. Verfahren nach Anspruch 3, **dadurch gekennzeichnet, dass** für den Endpunktnachweis die Reaktion mit einem Temperaturprofil von 80 °C, 5 Min. durchgeführt wird.

5. Verfahren zum Nachweis einer bakteriellen *Chlamydia trachomatis*-Infektion, **dadurch gekennzeichnet, dass** es das Nachweisverfahren nach Anspruch 2 umfasst.

6. Kit zum Nachweis von *Chlamydia trachomatis*-Infektion, **dadurch gekennzeichnet, dass** er einen Primersatz nach Anspruch 1 umfasst.

7. Kit nach Anspruch 6, **dadurch gekennzeichnet, dass** er die Amplifikationsprimer nach Anspruch 1 und nach Anspruch 2 enthält, wobei die Primer die folgenden Konzentrationen aufweisen: 0,13 µM F3, 0,13 µM B3, 1,06 µM FIP, 1,06 µM BIP, 0,27 µM LoopF, 0,27 µM LoopB; D-(+)-Trehalose-Dihydrat - 6 %; Fluoreszenzmarker, der mit doppelsträngiger DNA interagiert - EvaGreen ≤1X oder Fluoreszenzfarbstoff in einer Menge von <0,5 µl oder grüner Fluoreszenzfarbstoff in einer Menge von ≤1 µl oder Syto-13 ≤16 µM oder SYTO-82 ≤16 µM oder einen anderen Fluoreszenzfarbstoff, der mit doppelsträngiger DNA interagiert, in einer Konzentration, die die Amplifikationsreaktion nicht hemmt.

## Revendications

1. Ensemble d'amorces destinées à amplifier la séquence nucléotidique du gène *dnaK* de *Chlamydia trachomatis,* **caractérisé en ce qu'**il contient un ensemble d'amorces internes avec les séquences nucléotidiques a) et b) suivantes ainsi qu'un ensemble d'amorces externes contenant les séquences nucléotidiques c) et d) suivantes :
a) 5' GCGAGTTAGAGTTAAAGCCAAATGT 3'-(séquence nucléique SEQ ID N° : 3 ou sa séquence inverse ou complémentaire), liée ou non liée à partir de l'extrémité 3' par un pont TTTT à la séquence 5' TCTACTGAAATCAATCAGCCATT 3'-(séquence nucléique ou SEQ ID N° : 4 ou séquence inverse et séquence complémentaire)
b) 5' TCGAACACCTAGCTTCCTCTCT 3'- (séquence nucléique SEQ ID N° : 5 ou sa séquence inverse et sa séquence complémentaire), liée ou non liée à partir de l'extrémité 3' par un pont TTTT à la séquence 5' GAAGCGGACAATTTAGCAT 3'-(séquence nucléique SEQ ID N° : 6 ou sa séquence inverse et complémentaire)
c) séquence nucléique 5' AATAGAATTGTCTGGTGTATCG 3' SEQ ID N° : 1 ou sa séquence inverse et complémentaire, et
d) séquence nucléique 5' CTAGAAGAACATCATCAATGTCA 3' SEQ ID N° : 2 ou sa séquence inverse et complémentaire, dans lequel
il contient un ensemble de séquences d'amorces en boucle contenant des séquences nucléotidiques contenues dans ou complémentaires au gène *dnaK* de *Chlamydia trachomatis* SEQ ID N° : 7-5' GGTCCATTAGCGTCGATAGTGATG 3' et SEQ ID n° : 8 : 5' CCAAACAACCTTGTGCTCAGGCT 3' ou des séquences inverses et complémentaires à celles-ci.

2. Procédé de détection de bactéries *Chlamydia trachomatis,* **caractérisé en ce qu'**une région sélectionnée de la séquence nucléique du génome bactérien est amplifiée à l'aide de l'ensemble d'amorces tel que défini dans la revendication 1, le procédé d'amplification étant le procédé LAMP.

3. Procédé de détection de bactéries selon la revendication 2, **caractérisé en ce que** l'amplification est réalisée avec un profil de température de : 64 °C, 40 min.

4. Procédé selon la revendication 3, **caractérisé en ce que** pour la détection de point final une réaction est réalisée avec un profil de température de 80 °C, 5 min.

5. Procédé destiné à la détection d'une infection bactérienne à *Chlamydia trachomatis,* **caractérisé en ce qu'**il comprend le procédé de détection selon la revendication 2.

6. Kit destiné à la détection d'une infection à *Chlamydia trachomatis,* **caractérisé en ce qu'**il comprend un ensemble d'amorces tel que défini dans la revendication 1.

7. Kit selon la revendication 6, **caractérisé en ce qu'**il contient les amorces d'amplification telles que définies dans la revendication 1 et dans la revendication 2, dans lequel les amorces présentent les concentrations suivantes: 0,13 µM de F3, 0,13 µM de B3, 1,06 µM de FIP, 1,06 µM de BIP, 0,27 µM de LoopF, 0,27 µM LoopB ; D-(+)-dihydrate de tréhalose-6 %; marqueur fluorescent interagissant avec de l'ADN double brin-EvaGreen ≤ 1 X ou colorant fluorescent d'une quantité < 0,5 µl ou colorant fluorescent vert d'une quantité ≤ 1 µl ou Syto-13 ≤ 16 µM ou SYTO-82 ≤ 16 µM ou un autre colorant fluorescent interagissant avec de l'ADN double brin à une concentration qui n'inhibe pas la réaction d'amplification.
